# EUROPEAN PATENT APPLICATION

(11) **EP 1 935 424 A1**
(43) Date of publication of application: **25.06.2008**
(21) Application number: 06769370.5
(22) Date of filing: 23.06.2006
(51) Int. Cl.: A61K 31/64, A61K 31/426, A61K 31/155, A61P 3/10

(54) **PHARMACEUTICAL COMPOSITIONS COMPRISING COMBINED ANTIDIABETIC SUBSTANCES FOR USE IN DIABETES MELLITUS**

(30) Priority: 12.10.2005 MX PA05010977
(71) Applicant: ESPINOZA ABDALA, Leopoldo de Jesús, C.P. 45110 Zapopan Jalisco (MX); WORLD-TRADE IMPORT-EXPORT, WTIE, A.G., 6302 Zug (CH)
(72) Inventor: GARCÍA ARMENTA, María Elena, C.P. 45020 Guadalajara, Jalisco, México (MX); ÁLVAREZ OCHOA, Víctor Guillermo, C.P. 45222 Zapopan, Jalisco, México (MX); SANTOS MURILLO, Josefina, C.P. 44600 Zapopan, Jalisco, México (MX)
(74) Representative: Carvajal y Urquijo, Isabel
(86) International application number: PCT/MX2006/000058
(87) International publication number: WO 2007/043853

(57) **Abstract**

The present invention relates to the pharmaceutical industry in general and, in particular, to the pharmaceutical industry involved in the preparation of medicaments for controlling diabetes. More specifically, the invention relates to a composition comprising different types of antidiabetic substances in combination. The composition is **characterized in that** the aforementioned combination or association consists of two or three antidiabetic substances that are selected from the group containing: sulfonylureas, meglitinides, biguanides or inhibitors of alpha-glucosidase with thiazolidinediones for the treatment of type 2 diabetes mellitus in novel formulations and wherein the sulfonylureas are selected from the group containing glipizide, glyburide, glimepiride; meglitinides, repaglinide, nateglinide, thiazolidinediones or a combination of same; biguanides such as metformin; inhibitors of alpha-glucosidase known as acarbose, miglitol associated or combined with thiazolidinediones known as pioglitazone, rosiglitazone, troglitazone, such as a base or a physiologically-accepted salt.

## Description

### FIELD OF THE INVENTION

The present invention relates to the pharmaceutical industry in general and, in particular, to the pharmaceutical industry involved in the preparation of medicaments for controlling diabetes. More specifically, the invention relates to a composition comprising a combination of different types of antidiabetic substances.

### BACKGROUND OF THE INVENTION

Diabetes mellitus is a metabolic syndrome characterized by hyperglycemia which results from a defective secretion of insulin, a defective action of insulin or both. Chronic hyperglycemia from DM has been associated with long-term damage, dysfunction or failure of several organs, especially eyes, kidney, nerves, heart and blood vessels. Diabetes mellitus can be classified into:
• Type 1 DM, which could be mediated by immune process, or idiopathic. Diabetes mediated by immune process occurs as a consequence of an autoimmune destruction of pancreatic beta cells and it accounts for most of type 1 DM cases. Idiopathic diabetes is of unknown etiology and only a minority of patients suffering type 1 diabetes is in this category, most of them from African or Asiatic background. Its pharmacological treatment is with insulin.
• Type 2 DM represents 90-95% of all the cases of Diabetes Mellitus. Usually it began in adult life, after 40 years of life although it can occur at any age. It is characterized by resistance to insulin usually associated to a relative deficit of insulin. It can vary from the prevalence of resistance to insulin with a relative deficit of insulin to a prevalence of the deficit in insulin secretion with resistance to insulin. The risk of developing this form of diabetes increases with age, weight and lack of physical activity. It occurs more frequently in women with past gestational diabetes and in individuals suffering hypertension or dyslipemia. They do not require insulin to survive, although they might need insulin to achieve glycemic control. Oral hypoglycemiants are the indicated drugs in the treatment of type 2 DM.
• Other specific types: genetic defects affecting β cells function or insulin action, associated to exocrine pancreas disorders, associated to endocrinopathies, drug-induced, associated to infections and associated to genetic syndromes; and gestational Diabetes.
DM is a chronic disease with a high prevalence, in the year 2000 its prevalence was of 171.000.000 worldwide, and it is estimated that it will be of about 366.000.000 in the year 2030. In Mexico, its prevalence in the year 2000 was of 2.179.000 and it is estimated that in the year 2030 it will be of about 6.130.000, according to data from WHO.
In Mexico DM is the leading cause of external consultation demand in public and private health institutions and one of the main reasons for hospitalization. It's greater in women than in men. The life expectancy of a diabetic individual is two thirds of that expected; patients suffering from chronic complications have twice as much probabilities of dying than the general population.
Therefore, diabetes mellitus (DM) is a major problem of public health in Mexico. The tendency to the increase sharpens in 1988. In the last five years it became the first cause of death with 11% of total deceases in both sexes and 14% of these deceases are women. According to data from diabetes statistics in Mexico, the causes of death are DM complications:

| DM complications | IDC* 1997 % | 2001 % |
|---|---|---|
| Coma | 3.9 | 4.5 |
| Ketoacidosis | 5.2 | 3.2 |
| Renal complications | 30.0 | 30.4 |
| Ophtalmic complications | 0.0 | 0.0 |
| Neurological complications | 0.3 | 0.2 |
| Peripheral circulation complications | 1.4 | 1.3 |
| Other specified complications | 20.7 | 17.6 |
| Multiple complications | 21.5 | 24.4 |
| Non-specific complications | 2.8 | 3.6 |
| Without mention of complications | 14.1 | 14.9 |

| | | |
|---|---|---|
| * IDC = International Disease Classification • Source: CEMECE, 2002 | | |

It is important to observe that renal complications of DM are the major cause of death in these patients, for this reason, the therapy for DM must be aimed to glucose control and prevention of complications.
Chronic complications can occur after having high glucose levels in blood for extended periods of time (years) and they are: diabetic retinopathy, diabetic nephropathy, diabetic neuropathy and cardiovascular complications.
As mentioned before, the fundamental aspects in the treatment are: diet and weight control, exercise, and therapy using medicaments, in any of the different pharmaceutical forms.
Solid pharmaceutical forms are preparations that contain the active principle(s) and additives, generally disc-shaped, scored or non-scored, and of variable size obtained by compressing powders or granules into tablets, capsules, patches, pessaries, beads, suppositories, troches or lozenges.
A solution is a clear and homogeneous liquid preparation obtained by dissolving the active principle(s) and additives in water, and which is employed for external or internal use.
Suspensions are those preparations consisting in a dispersed system, comprising two phases, which contain the active principle(s) and additives. One of the phases, the continuous or external phase is generally a liquid or a semisolid and the dispersed or internal phase is comprised of non-soluble solids (active principle(s)) but dispersable in the external phase.
An emulsion is a heterogeneous system comprised of two liquids non-miscible to each other, wherein the dispersed phase is comprised of small droplets distributed in the vehicle in which they are immiscible. The active principle(s) can be in the external or in the internal phase.

### Sulfonylureas

All drugs belonging to this group are sulfamides derivatives, in which the sulfonylurea moiety constitutes the essential group of the hypoglycemiant action and it can be represented by formula (I).

Several substitutions on the benzene ring and on the urea group, have given place to compounds (II), which potency and pharmacokinetic properties differ, especially the duration of action and the principal metabolic route, as shown below:

| General formula | 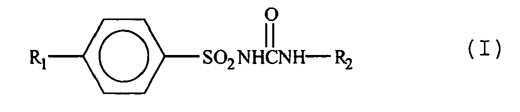 | |
|---|---|---|
| | R₁ | R₂ |
| **First generation** | | |
| Tolbutamide | H₃C- | -C₄H₉ |
| Chlorpropamide | Cl- | -C₃H₇ |
| Tolazamide | H₃C- | 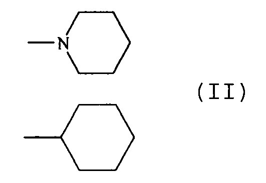 |
| Acetohexamide | H₃CCO- | |

| **Second generation** | | |
|---|---|---|
| Glibenclamide | 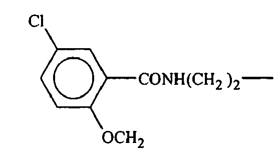 | 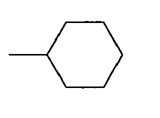 |
| Glipizide | 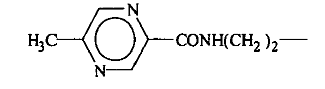 | 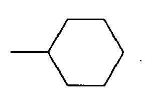 |
| Glicazide | H₃C- | 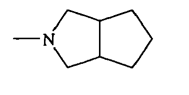 |
| Glimepiride | 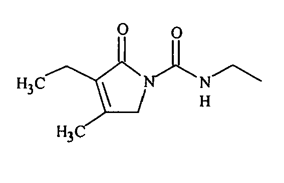 | 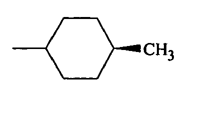 |

**Sulfonylureas: Pharmacokinetc Properties**

| Medicament | Clearance | Action duration | Daily doses (mg/day) |
|---|---|---|---|
| Chlorpropamide | Hepatic and renal | 24-72 | 100-500 (once daily) |
| Glibenclamide | Hepatic | 12-16 | 2.5-20 (1-2 times daily) |
| Glimepiride | Hepatic and renal | 24 | 1-4 (once daily) |
| Glicazide | Hepatic | 12-13 | 40-320 (once daily) |
| Glipizide | Hepatic | 5-10 | 2.5-30 (once daily) |
| Gliquidone | Hepatic | 2-4 | 45-280 (1-3 times daily) |

Sulfonylureas are known by its hypoglycemiant activity, they are considered the drugs of election in patients with type 2 DM of relatively recent onset (< 5 years), having a endogenous production of insulin, non obese and that do not respond appropriately to the dietetic treatment.

Sulfonylureas stimulate beta cells of the pancreas so that they liberate more insulin. They have been used since 1950. Chlorpropamide is the only first generation sulfonylurea still in use. Second generation sulfonylureas are: glipizide, gliburide (glibenclamide), glimepiride*. This medicaments are administered once or twice daily before meals. All sulfonylureas produce similar effects on blood glucose level, but they differ in the side effects, in how frequently they are administered and in the interactions with other medicaments.

### Meglitinides

Meglitinide is the nonsulfonylic sequence from sulfonylurea glibenclamide; this fraction is able to bind on its own to subunit SUR1 of ATP-dependent K⁺ channel. From meglitinide two analogs are derived: repaglinide, which is an active S enantiomer of a derivative from carbamoyl-methylbenzoic acid, and nateglinide which is a D-phenylalanine derivative. Their chemical structures are represented in figure (III):

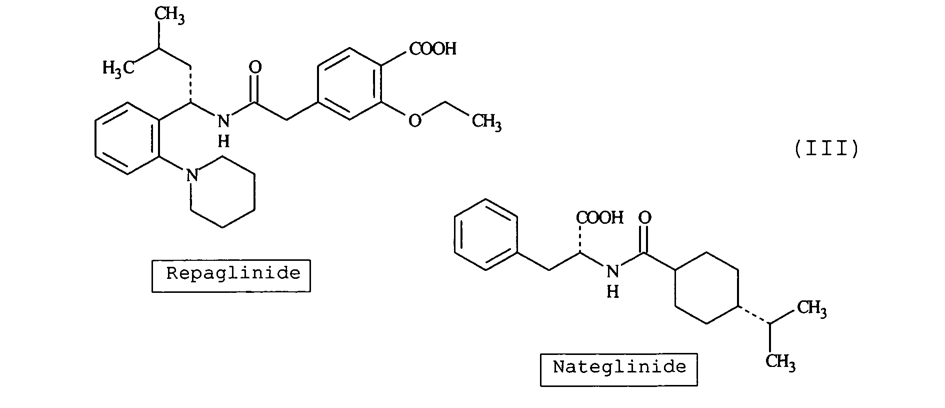

Compounds of formula (III) are known by their antidiabetic activity, designed to normalize post-prandial glucose levels in patientes having type 2 diabetes. They act as insulin secretagogues in pancreatic β cells, of short action. Like sulfonylureas, they stimulate insulin release by closing the ATP-dependant potassium channels in pancreatic β cells. They are indicated in patients suffering from type 2 diabetes whose hyperglycemia can not be controlled with diet, weight reduction and exercise.

They are administered before each meal. Since sulfonylureas and meglitinides stimulate insulin release ter is a possibility that they provoke hypoglycemia.

### Biguanides

Biguanides are drugs derived from guanidine. They were introduced into type 2 DM treatment in the decade of 1950-1960. The most common in the market is Metformin, whose chemical structure is represented in formula (IV).

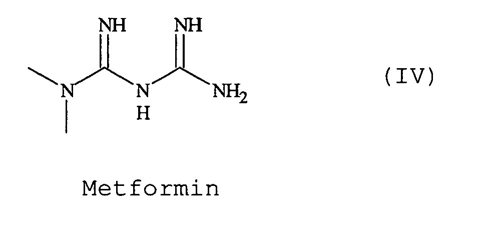

Compound of formula (IV) is known by its pharmacological activity as an anti-hyperglycemiant drug, non-hypoglycemiant, which reduces basal and post-prandial hyperglycemia. It lowers blood glucose mainly by reducing hepatic gluconeogenesis, that is, by diminishing the amount of glucose produced by the liver due to a higher utilization of glucose by peripheral tissues making the muscular tissue more sensitive to insulin such that glucose can be absorbed and a reduction in oral absorption of saccharides. Usually, they are administered twice daily. One of the possible side effects is diarrhea.

### Alpha glucosidases inhibitors

This group of drugs has been developed with the objective of delaying digestion and absorption of sucrose and complex carbohydrates, in order to improve post-prandial hyperglycemia in diabetic patients. In this group are included acarbose, miglitol and voglibose, which are represented by formula (V):

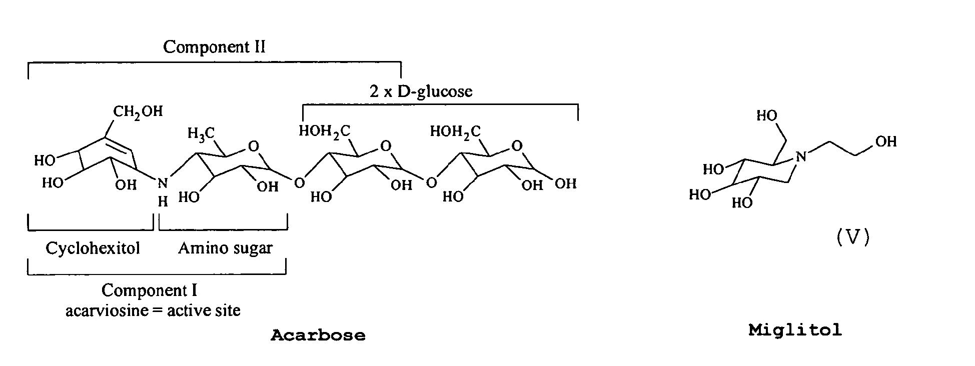

They are indicated as adjuvant to other treatments, especially in obese patients suffering from mild diabetes, when there is not adherence to a diet, or as monotherapy in cases of post-prandial hyperglycemia. Acarbose is the most used.

They help the body to reduce glucose levels in blood by blocking the degradation of starches such as bread, potato and pastas in the intestine. They also reduce the rate of digestion of certain sugars such as table sugar. Their action reduces the increase of glucose level in blood after a meal. They should be taken with the first bite of a meal.

### Thiazolidinediones or glitazones

They are a novel group of hypoglycemiants, which are characterized by sensitizing or increasing insulin action without increasing its secretion; thereby they are useful in situations where peripheral resistance to insulin has developed. Among this group troglitazone, pioglitazone and rosiglitazone are included, which are represented in figure (VI):

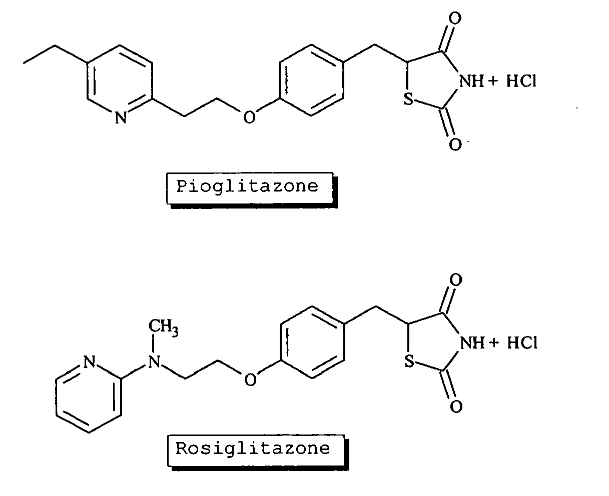

Thiazolidinediones act improving insulin sensitivity in patients who had developed resistance to insulin, decreasing glycemia, insulin and HbA1c levels, without producing hypoglycemia. They are indicated in patients with type 2 DM having resistance to insulin or in patients mismanaged with other forms of therapy. They can be used in monotherapy or in association with insulin and other hypoglycemiants. They are administered one or two times daily with meals.

Combination therapy with oral medicaments: It has been described in the literature that management of diabetes mellitus should be done by combining medicaments since this will help to improve control of glucose in blood, since if only one medicament is administered, the desired results are not obtained.

We realize a series of studies to evaluate the combinations that are better tolerated, effective and with minor side effects for the treatment of the type 2 diabetes mellitus.

### SUMMARY OF THE INVENTION

Therefore, the present invention provides new formulations comprising: the association or combination of antidiabetic substances such as sulfonylureas, meglitinides, biguanides or inhibitors of alpha-glucuronidase with thiazolidinediones for the treatment of type 2 diabetes mellitus.

This invention refers to formulations in different pharmaceutical forms of pharmaceutical compositions comprising antidiabetic substances combined for use in diabetes mellitus.

It also comprises, more specifically the association or combination or antidiabetic substances such as sulfonylureas, meglitinides, biguanides or alpha-glucuronidase inhibitors with thiazolidinediones for treatment of diabetes mellitus type 2.

In new formulations comprising: (a) one or more anti-adhering agents, (b) one or more disintegrating agents, (c) one or more binding agents, (d) one or more lubricants, (e) one or more diluting agents, (f) one or more solvents, (g) one or more solubilizing agents, (h) one or more sweeteners, (i) one or more flavoring and/or perfuming agents, (j) one or more viscosity agents, (k) one or more antimicrobial agents, (l) one or more surfactants, (m) one or more antioxidants, (n) one or more emulsifiers, and (o) any other additive that facilitates formulation.

Also, in another of its aspects, the present invention provides a novel method of treating Diabetes Mellitus, since the combination of these produce a sinergistic effect, and also the dosage of each of these components is lower, thereby preventing side effects that might occur when they are administered independently.

This invention is the result of a series of studies carried out to evaluate those combinations that are well tolerated, effective and with fewer side effects for the treatment of diabetes mellitus type 2.

### DETAILED DESCRIPTION OF THE INVENTION

We performed different assays comparing combinations of sulfonylureas such as glipizide, gliburide and glimepiride, meglitinides such as repaglinide, nateglinide, biguanides such as metformin, alpha-glucoronidase inhibitors such as acarbose, miglitol with thiazolidinediones such as pioglitazone, rosiglitazone, troglitazone, in order to evaluate which combination is more suitable in terms of effectiveness, as well as fewer side effects.

### EXAMPLE 1

We carried a study to evaluate the effectiveness and tolerability of a combination of sulfonylurea with a thiazolidinedione such as glimepiride/pioglitazone in treating patients suffering from diabetes mellitus type 2. The study was a 16 weeks, double blinded study, that included patients with stable regimen of a sulfonylurea for ≥ 30 days, with glycosylated hemoglobin ≥ 8.0%, patients were randomized to receive Group 1 the once a day combination, Group 2 was designated to receive Pioglitazone alone once a day.

### Results

Patients administered with the combination of glimepiride/pioglitazone exhibited a significant reduction of basal levels (P = <0.05) in glycosylated hemoglobin compared to the group of pioglitazone alone. Glycosylated Hemoglobin diminished to 0.9% (95% Confidence Interval): 0.06% to 1.2% with the combination. The group who received the combination had a percentage of reduction in triglyceride levels of (26%, 95% IC, 16% to 36%) and an increase in the levels of high density lipids (13%, IC, 8% to 18%) compared to group that received pioglitazone alone. In the group receiving the combination there is a small but significant increase in the levels of low-density lipoproteins.

### Conclusions

In patients with diabetes mellitus type 2, the combination of glimepiride/pioglitazone significantly improves glycosylated Hemoglobin and glucose plasma levels with beneficial effects on cholesterol and triglycerides levels.

### EXAMPLE 2

This study evaluates the effectiveness and safety of a combination of the glucosidase inhibitor acarbose and a thiazolidinedione, pioglitazone, in 30 patients suffering from diabetes mellitus type 2 having a poor glycemic control with the administration of acarbose alone. Patients exhibited a glycosylated hemoglobin between 7.0 to 12.0% and plasma glucose was 7.8 mmol/1 or more. Patients were treated with the combination once a day for 16 weeks.

### Results

The reduction in glycosylated hemoglobin on week 16 of treatment was 0.8% (7.8% basal and 7.1% on week 16 (P = 0.01). The correlation between the reduction in glycosylated hemoglobin on week 16 of treatment and the variable characteristics of patients was also analyzed. A correlation to the levels of leptine (p = -0.5632) was found, 10 patients exhibited adverse reaction such as edema, hypoglycemia, increased creatine phosphokinase (CK) which were mild in severity. The combination of pioglitazone with acarbose in diabetic patients with poor glycemic control was effective, although the number of adverse events was high.

### EXAMPLE 3

It is suggested by the complementary mechanism of action that a combination of pioglitazone and metformin could have clinically beneficial effects on the treatment of patients with diabetes mellitus type 2. It was a double-blinded study with a 16 weeks follow-up. Patients included in this study were those with a poor control of diabetes mellitus (Glycosylated hemoglobin ≥ 8.0% who previously received an stable dosing of metformin for 30 days or more. Patients having diabetic retinopathy, nephropathy or neuropathy; hepatic or renal failure or unstable cardiovascular or cerebrovascular conditions were excluded. Patients were randomized to receive the once a day combination or placebo + metformin.

### Results

100 patients were randomized for treatment (50 with pioglitazone/metformin) and (50 placebo/Metformin). All of these 100 patients concluded the study. Patients' average age was 57 years. Patients who received the combination of pioglitazone/metformin exhibited a statistically significant reduction in glycosylated hemoglobin (-0.83%) and plasma glucose levels of (-37.8 mg/dL) compared to placebo + metformin (P ≤ 0.05). The reduction in glucose levels occurred during week 4 of treatment. The combination of pioglitazone/metformin achieved significant percent changes in triglycerides (-18.2%) and high density lipoproteins (+ 8.7%) compared to the group that received placebo/metformin (P<0.05). The increase in percentage was observed in low-density lipoproteins: 7.7% in the group receiving the combination pioglitazone/metformin; 11.9% in the group administered with placebo/metformin without significant differences in the groups.

### Conclusions

Patients who received the combination pioglitazone/metformina exhibit significant improvement in glycosylated hemoglobin and in plasma glucose levels without evidence of drug-induced hepatic toxicity.

The present invention is characterized in that the combined drugs therein can be found as it anhydrous, monohydrated or dehydrated base or as a physiologically acceptable salt.

The pharmaceutical form can contain a series of additives or excipients such as, for example corn starch, alginic acid, celluloses and their derivatives, povidone, sodium crosscarmellose, sodium starch glycolate, among others, with sodium starch glycolate being preferred. The disintegrating agent can be present from 0.1 % to 25.0 %.

Lubricating agents such as stearic acid, magnesium stearate, talc, among others, with magnesium stearate being preferred. The lubricating agent can be present in a proportion from 0.01% to 10.0 %.

Diluents such as lactose, mannitol, calcium phosphate, microcrystalline cellulose, calcium sulfate, sugar for compression, corn starch, pre-gelatinized starch, among others, with microcrystalline cellulose being preferred. The diluent can be present in a proportion from 5% to 99%.

Coatings such as methacrylates, polyvinyl alcohol, cellulose derivatives, a blend of polymers and polysaccharides for coating by film formation with aqueous or organic solvents, with coloring agents, flavoring agents, sugars and any other ingredient that is used for film forming, coating and dragee making applications such as talc, titanium dioxide, among others. The coating does not contain any active principle. Capsules can be of hard or soft gelatin, with hard gelatin capsules being preferred.

Polar and non-polar solvents such as: water, ethyl alcohol, isopropyl alcohol, isopropyl myristate, polyoxypropylenes, glycofurol, propylene glycol, polyethylene glycol, glycerol, 70% sorbitol, dimethylacetamide, mineral oil, petrolatum, lanoline, vegetable waxes, animal waxes, vegetable oils such as olive oil, cottonseed oil, corn oil, among others, with ethyl alcohol, glycofurol and polar solvents such as water being the more preferred to be used. The final formulation can contain from 1% to 95% w/v of the dissolvent.

Solubilizing agents such as povidone, cyclodextrins, N-methylglucamine, lecithin, monoethanolamine, glycine, benzyl benzoate, poloxamers, polyoxyethylene alkyl ethers, among others. The solubilizing agent can be present in a proportion from 0.0001 to 50.0 %.

Viscosifying agents such as acacia, agar, alginic acid, povidone, hydroxypropylmethylcellulose, sodium carboxymethylcellulose, calcium carboxymethylcellulose, pectin, gelatin, guar gum, xanthan gum, carbomer, bentonite, hydroxypropylcellulose, among others. Final formulation can contain from 0.0001% to 10.0% w/v of the viscosifying agent.

Flavoring agents, such as menthol, vanillin, cinnamon, sorbitol, citric acid, cherry flavor, orange flavor, peach flavor, grape flavor, strawberry flavor, among others. The flavoring agent can be present in a proportion from 0.0001% to 5.0% w/v.

Sweetening agents such as aspartame, acesulfame k, alitame, dextrose, fructose, glucose, mannitol, sorbitol, sugar, sucrose, among others. The sweetening agent can be present in a proportion from 10.0% to 60.0% w/v.

Antimicrobial agents such as sorbic acid; potassium benzoate; sodium benzoate; chlorobutanol; methyl p-hydroxybenzoate; propyl p-hydroxybenzoate; benzalkonium chloride, benzoic acid, benzyl alcohol, thimerosal, bronopol, monothioglycerol, among others. The antimicrobial agent can be present in a proportion from 0.0001% to 5.0% w/v.

One or more surfactants such as sodium laurate, sodium oleate, sodium lauryl sulfate, sodium cholate, sodium deoxycholate, sodium diamylsulfosuccinate, sodium dioctylsulfosuccinate, poloxamers, lecithin, tetradecyltrimethylammonium bromide, hexadecylpyridinium chloride, polysorbate 20, polysorbate 60, among others. The surfactant can be present in a proportion from 0.0001% to 30%.

Antioxidants such as disodium edetate (EDTA), Butylhydroxytoluene (BHT), tocopherols, sodium bisulfite or sodium metabisulfite, gallic acid, propylgallate, ascorbyl palmitate, among others. The antioxidant can be present in a proportion from 0.0001% to 20.0%.

Emulsifying agents such as Arabic gum, tragacanth gum, alginates, chondrus, pectin, aluminum silicate, bentonite, aluminum magnesium silicate, sodium dodecylsulfate, benzalconium chloride, polyoxyethylene sorbitan monostereate, polyethylene glycol 400 monostearate, ethylene glycol distearate, polyethylene glycol 400, ethylene glycol distearate, sorbitan tristearate, sorbitan monopalmitate, diethylene glycol monostearate, sodium oleate, potassium oleate, sodium lauryl sulfate, among others, with sorbitan monostearate and polyoxyethylene sorbitan monostearate being preferred. The emulsifying agent can be present from 0.0001% to 10%.

The formulation can also contain other components such as a flocculating agent like sodium chloride or potassium chloride; tonicity agents such as sodium chloride, potassium chloride, dextrose, mannitol, among others. A pH buffering system such as phosphates, citrates, carbonates, bicarbonates, acetates, lactates, among others. Alcalinizing or acidifying agents such as sodium hydroxide, sodium bicarbonate, monoethanolamine, triethanolamine, hydrochloric acid, citric acid, acetic acid, among others. Binding agents such as polyvidone, tragacanth, acacia, starch, methylcellulose, among others; anti-adhering agents such as colloidal silicon dioxide, calcium sulfate, calcium chloride, talc, and cornstarch, among others.

The formulations can be contained in a blisterpack made of PVC (polyvinyl chloride) film with a thickness from 200 µ to 250 µ, which optionally can have a coating of PVDC (polyvinylidene chloride) with a weight from 25 g/m² to 120 g/m², and bonded with aluminum foil or in cellopolyal film. In containers having suitable capacity varying from 1 ml to 500 ml elaborated from high and/or low density polyethylene, polyethylene terephthalate, polyvinyl chloride, polypropylene, polystyrene, borosilicate glass (Type I glass), or soda-lime glass type II and III and type IV glass, among others, colored or non colored.

The cap may be of the following types: tamperproof, threaded, cap-to-cap, child proof, elaborated from high and/or low density polyethylene, polyethylene terephthalate, polyvinyl chloride, polypropylene, polystyrene, chlorobutyl, colored or non colored. Also, dosing auxiliaries, such as dosing spoons, metering cups, pipettes, small syringes, inserts elaborated from materials such as, *inter alia,* high and/or low density polyethylene, polyethylene terephthalate, polyvinyl chloride, polypropylene, polystyrene, colored or non-colored, are included. For the solution, suspension and/or emulsion a bottle and cap made of high density polyethylene or type I glass containers having a capacity of 1 to 150 ml are preferred and for solid pharmaceutical it is preferred a blisterpack made of polyvinyl chloride coated with polyvinylidene chloride. The elaboration of the different pharmaceutical forms is carried out mixing the active principles with the corresponding additives, in the proper concentrations.

Generally, sulfonylureas are well absorbed orally. Its absorption is decreased in hyperglycemia situations. They bind to plasma proteins in 90-99%, being able to produce interactions with many drugs by inhibiting the binding thereof to said proteins. The advantage of new sulfonylureas in relation to the older ones, is that their binding to plasma proteins is of the non-ionic type, and therefore they are less susceptible to drug interactions due to plasma protein binding inhibition, than that of first generation sulfonylureas by drugs such as warfarin or salycilates, which gives place to hypoglucemic symptoms. Sulfonylureas differ basically in their pharmacokinetics, especially in the duration of their action and in the main metabolic route. Regarding the duration of their action, it should be considered that the longer said action is, the better, since less daily doses are required to be administered, but on the contrary, should a hypoglycemic episode occurs, it will be last more.

Chlorpropamide has a prolonged half-life (24 to 48 hours). Second generation sulfonylureas are considered 100 times more potent than first generation sulfonylureas. Their half-lives are short (1.5 to 5 hours) but their hypoglucemiant effects continued for 12 to 24 hours, and frequently it is possible to administer them in a once a day regime. Most of sulfonylureas are metabolized through liver and others are eliminated without being metabolized by renal route (such as chlorpropamide which is eliminated in about 20% without being metabolized).

Repaglinide is rapidly absorbed from gastrointestinal tract; plasma peak is obtained 1 hour later following administration and is eliminated between 4-6 hours later. The clearance half-life in plasma is of about 1 hour due to its rapid metabolism by cytochrome P450 and by glucuronidases. It binds to plasma proteins (albumin) in more than 98%. Hepatic failure could increase its concentration, but renal failure from mild to moderated does not alter drug clearance. In patients with type 2 diabetes, the insulinotropic response to a meal occurs within 30 minutes after taking an oral dose of repaglinide, producing a hypoglycemiant effect throughout the meal. Low plasma concentrations are observed at 4 hours after administration.

Nateglinide is rapidly absorbed, with a bioavailability greater than 70% and tₘₐₓ of 1 hour. It should be administered between 1 to 30 minutes before meals, at doses from 60 to 120 mg, generally three times a day. Maximum daily doses should not exceed 180 mg before meals. It binds to plasma proteins in 97%. Clearance half-life is of about 1.5 hours. It is highly metabolized in liver by isoenzymes CYP3A4 and CYP2C9, with metabolites being eliminated through urine. Mild to moderate hepatic cirrhosis could reduce the clearance. About 16% of the administered dose is excreted through kidney as non-metabolized drug.

Metformin is slowly and incompletely absorbed by gastrointestinal tract; its absorption is completed in about 6 hours and without undergoing hepatic metabolization. It is excreted by renal route (90% of an oral dose in 12 hours). It exhibits poor union to plasma proteins. Metformin has a half-life of plasmatic clearance of about 2-4 hours, thereby it should be administered 2-3 times a day. It is recommended that metformin should be administered with meals to prevent gastrointestinal side effects.

Acarbose is poorly absorbed orally < 2%. It should be taken with each of the meals and in order to achieve maximal efficiency it should be chewed during the first 15 minutes when the patient stars to feed. The treatment begins at low doses, 25 mg/8 hours, and it can be increased, if needed, up to 200 mg/8 hours.

Rosiglitazone is well absorbed orally with a tₘₐₓ of 1 hour and a bioavailability of 99%. It binds to plasma proteins in 99.8% (mainly albumin); it is metabolized in liver by cytochrome P450 isoenzymes 2C8 and 2C9, and is eliminated in 64% in urine and in 23% in feces. Its clearance half-life is of about 3-4 hours. Rosiglitazone is used in doses of 4-8 mg/day, distributed in two portions, taken with or without food.

Pioglitazone has a bioavailability close to 80% with a tₘₐₓ of 2 hours. It binds to plasma proteins in more than 99%. It is metabolized by CYP3A4 and CYP2C9, giving rise to three active metabolites. Its clearance half-life is of about 5-6 hours for the parent compound and of about 16-24 hours for the metabolites.

For Diabetes Mellitus treatment it is recommended to use 15 mg every 12 to 24 hours, as considered by the physician.

Accordingly, the present invention provides a pharmaceutical formulation, in solid form, in solution, in suspension, or emulsion, which provides 15 mg to 30 mg of Pioglitazone in suitable doses.

### EXAMPLES OF DIFFERENT PHARMACEUTICAL FORMS

The following non-limiting examples will assist to illustrate the present invention:

### EXAMPLE 1

| | | |
|---|---|---|
| 1) | Pioglitazone | 30.00 % w/w |
| 2) | Glimepiride | 4.00 % w/w |
| 3) | Microcrystalline Cellulose pH 101 | 59.00 % w/w |
| 4) | Sodium starch glycolate | 3.00 %.w/w |
| 5) | Polyvidone | 3.00 % w/w |
| 6) | Magnesium stearate | 1.00 % w/w |

Tablet elaboration is as follows: Pioglitazone together with Glimepiride and Sodium starch glycolate are mixed, then Polividone and Microcrystalline Cellulose pH 101 are added. Finally, magnesium stearate is added and all the ingredients are mixed. Proceed to tableting.

### EXAMPLE 2

| | | |
|---|---|---|
| 1) | Pioglitazone | 15.00 % w/w |
| 2) | Metformin | 60.00 % w/w |
| 3) | Pre-gelatinized starch | 19.00% w/w |
| 4) | Sodium starch glycolate | 2.50 % w/w |
| 5) | Polividone | 2.50% w/w |
| 6) | Magnesium stearate | 1.00 % w/w |

Powder making is carried out as follows: Pioglitazone together with Metformin and Sodium starch glycolate are mixed, then Polividone is added. Then, pre-gelatinized Starch is added and mixed. Finally, magnesium stearate is added and all the ingredients are mixed. Proceed to tableting.

The invention has been described sufficiently as to allow that a person of ordinary skill in the art could realize and obtain the results mentioned in the present invention. However, any person of ordinary skill in the art to which the present invention pertains could be able to make modifications not disclosed in the present application, nonetheless, if for the application of these modifications in a given structure or in the manufacturing process thereof, the subject matter claimed in the following claims is required, said structures should be embraced within the scope of the invention.

## Claims

1. Pharmaceutical compositions comprising combined antidiabetic substances for use in diabetes mellitus, wherein said combination or association consists in two or three antidiabetic substances selected from the group consisting of: sulfonylureas, meglitinides, biguanides or alpha glucosidase inhibitors with thiazolidinediones for treatment of diabetes mellitus type 2 in novel formulations.

2. Pharmaceutical compositions comprising combined antidiabetic substances for use in diabetes mellitus, as claimed in claim 1 wherein said sulfonylureas are selected from the group consisting of glipizide, gliburide, glimepiride; meglitinides, repaglinide, nateglinide, thiazolidinediones or a combination thereof; biguanides such as metformin; alpha-glucosidase inhibitors known as acarbose, miglitol in association or combination with thiazolidinediones known as pioglitazone, rosiglitazone, troglitazone, as the base or a physiologically acceptable salt.

3. Pharmaceutical compositions comprising combined antidiabetic substances for use in diabetes mellitus, as claimed in claim 2, wherein said sulfonylureas are selected from the group consisting of glimepiride and thiazolidinediones and said glimeride is present in a proportion from 2 to 4 mg and said pioglitazone is present in a proportion from 15 to 30 mg.

4. - The composition as claimed in claim 1, wherein said solid pharmaceutical form contains one or more disintegrating agents desintegrantes such as corn starch, alginic acid, celluloses and their derivatives, povidone, sodium crosscarmellose, sodium starch glycolate, among others. The disintegrating agent can be present from 0.1 % to 25.0%. Lubricating agents such as stearic acid, magnesium stearate, talc, among others. The lubricating agent can be present in a proportion from 0.01% to 10.0 %. Diluting agents such as lactose, mannitol, calcium phosphate, microcrystalline cellulose, calcium sulfate, sugar for compression, cornstarch, pre-gelatinized starch, among others. The diluting agent can be present in a proportion from 5% a 99%. Coatings such as methacrylates, polyvinyl alcohol, cellulose derivatives, a blend of polymers and polysaccharides for coating by film formation with aqueous or organic solvents, with coloring agents, flavoring agents, sugars and any other ingredient that is used for film forming, coating and dragee making applications such as talc, titanium dioxide, among others. Said coating does not contain any active principle. Capsules can be of hard or soft gelatin.

5. Pharmaceutical compositions comprising combined antidiabetic substances for use in diabetes mellitus, as claimed in claim 4, wherein said formulation is solid and comprises other components such as: binding agents like polyvidone, tragacanth, acacia, starch, methylcellulose, among others. Anti-adhering agents such as, colloidal silicon dioxide, calcium sulfate, calcium chloride, talc, corn starch, among others,

6. Pharmaceutical compositions comprising combined antidiabetic substances for use in diabetes mellitus, as claimed in any one of the preceding claims, wherein said pharmaceutical form in tablet or capsule form can be contained in a blisterpack made of PVC (polyvinyl chloride) film with a thickness from 200 µ to 250 µ, which optionally coated or uncoated with PVDC (polyvinylidene chloride) with a weight from 25 g/m² to 120 g/m², and bonded with aluminum foil or in cellopolyal film.

7. Pharmaceutical compositions comprising combined antidiabetic substances for use in diabetes mellitus, as claimed in any of claims 1 to 6, wherein said pharmaceutcal form in solution, suspension or emulsion form contains one or more polar and non-polar solvents such as son: : water, ethyl alcohol, isopropyl alcohol, isopropyl myristate, polyoxypropylenes, glycofurol, propylene glycol, polyethylene glycol, glycerol, 70% sorbitol, dimethylacetamide, mineral oil, petrolatum, lanoline, vegetable waxes, animal waxes, vegetable oils such as olive oil, cottonseed oil, corn oil, among others. The final formulation can contain from 1% to 95% of the dissolvent; solubilizing agents such as povidone, cyclodextrins, N-methylglucamine, lecithin, monoethanolamine, glycine, benzyl benzoate, poloxamers, polyoxyethylene alkyl ethers, among others; the solubilizing agent can be present in a proportion from 0.0001 to 50.0 %; viscosifying agents such as acacia, agar, alginic acid, povidone, hydroxypropylmethylcellulose, sodium carboxymethylcellulose, calcium carboxymethylcellulose, pectin, gelatin, guar gum, xanthan gum, carbomer, bentonite, hydroxypropylcellulose, among others; the final formulation can contain from 0.0001% to 10.0% w/v of the viscosifying agent; flavoring agents, such as menthol, vanillin, cinnamon, sorbitol, citric acid, cherry flavor, orange flavor, peach flavor, grape flavor, strawberry flavor, among others; the flavoring agent can be present in a proportion from 0.0001% to 5.0% w/v; Sweetening agents such as aspartame, acesulfame k, alitame, dextrose, fructose, glucose, mannitol, sorbitol, sugar, sucrose, among others; the sweetening agent can be present in a proportion from 10.% to 60.0% w/v; antimicrobial agents such as sorbic acid; potassium benzoate; sodium benzoate; chlorobutanol; methyl p-hydroxybenzoate; propyl p-hydroxybenzoate; benzalkonium chloride, benzoic acid, benzyl alcohol, thimerosal, bronopol, monothioglycerol, among others; the antimicrobial agent can be present in a proportion from 0.0001% to 5.0% w/v; one or more surfactants such as sodium laurate, sodium oleate, sodium lauryl sulfate, sodium cholate, sodium deoxycholate, sodium diamylsulfosuccinate, sodium dioctylsulfosuccinate, poloxamers, lecithin, tetradecyltrimethylammonium bromide, hexadecylpyridinium chloride, polysorbate 20, polysorbate 60, among others; the surfactant can be present in a proportion from 0.0001% to 30%; antioxidants such as disodium edetate (EDTA), Butylhydroxytoluene (BHT), tocopherols, sodium bisulfite or sodium metabisulfite, gallic acid, propylgallate, ascorbyl palmitate, among others; the antioxidant can be present in a proportion from 0.0001% to 20.0%; emulsifying agents such as Arabic gum, tragacanth gum, alginates, chondrus, pectin, aluminum silicate, bentonite, aluminum magnesium silicate, sodium dodecylsulfate, benzalkonium chloride, polyoxyethylene sorbitan monostereate, polyethylene glycol 400 monostearate, ethylene glycol distearate, polyethylene glycol 400, ethylene glycol distearate, sorbitan tristearate, sorbitan monopalmitate, diethylene glycol monostearate, sodium oleate, potassium oleate, sodium lauryl sulfate, among others, with sorbitan monostearate and polyoxyethylene sorbitan monostearate being preferred. The emulsifying agent can be present from 0.0001% to 10%.

8. Pharmaceutical compositions comprising combined antidiabetic substances for use in diabetes mellitus, as claimed in claim 7, further comprising other components such as: a flocculating agent like sodium chloride or potassium chloride; tonicity agents such as sodium chloride, potassium chloride, dextrose, mannitol, among others. A pH buffering system such as phosphates, citrates, carbonates, bicarbonates, acetates, lactates, among others. Alcalinizing or acidifying agents such as sodium hydroxide, sodium bicarbonate, monoethanolamine, triethanolamine, hydrochloric acid, citric acid, acetic acid, among others.
